# EUROPEAN PATENT APPLICATION

(11) **EP 3 189 871 A1**
(43) Date of publication of application: **12.07.2017**
(21) Application number: 15838333.1
(22) Date of filing: 31.08.2015
(51) Int. Cl.: A61M 25/10

(54) **CATHETER AND METHOD FOR MANUFACTURING SAME**

(30) Priority: 04.09.2014 JP 2014180092
(71) Applicant: Terumo Kabushiki Kaisha, Tokyo 151-0072 (JP)
(72) Inventor: MAEDA, Naoyuki, Fujinomiya-shi, Shizuoka 418-0015 (JP)
(74) Representative: Casalonga
(86) International application number: PCT/JP2015/074625
(87) International publication number: WO 2016/035741

(57) **Abstract**

In a catheter (10), a tubularly net-shaped reinforcement member (28) is disposed between an inner layer (24) and an outer layer (26) configuring a balloon (14). The reinforcement member (28) has a first thread (29) formed of a high-strength fiber. Both end portions of the reinforcement member (28) in an axial direction are respectively provided with ring-shaped inflation restriction portions (36) welded to the first thread (29). The inflation restriction portions (36) respectively restrict inflation of both the end portions of the reinforcement member (28) in a circumferential direction.

## Description

### Technical Field

The present invention relates to a catheter including a balloon reinforced with a reinforcement member, and a method of manufacturing the same.

### Background Art

Recently, for example, in treatment of acute myocardial infarction and angina pectoris, percutaneous coronary intervention (percutaneous transluminal coronary angioplasty) in which a blood flow is improved by widening a lesion (stenosed portion) of the coronary artery with a balloon catheter has been performed (for example, refer to JP-T-2008-501408). Treatment using a balloon catheter is also performed so as to improve a lesion formed inside other blood vessels, the bile duct, the trachea, the esophagus, the urethra, and other body lumens.

Generally, a balloon catheter is configured to include a long shaft, and a balloon which is provided on the distal side of the shaft and inflates in the radial direction. The balloon catheter is delivered to a stenosed portion in a body after a preceding guide wire is inserted through. In a state where the balloon is disposed at the target stenosed portion, the balloon is inflated by pressure-feeding a inflation fluid into the balloon, and thus, the stenosed portion can be widened.

In order to effectively treat a lesion, the balloon used in the balloon catheter is required to have sufficient strength so as to have a desired balloon shape when being maximally inflated, and to widen the lesion. Therefore, in the related art, in order to apply high-pressure resistance, low compliance properties, and the like to a balloon, a configuration in which a net-shaped reinforcement member is provided in a wall configuring the balloon has been proposed (for example, refer to JP-T-2008-501408).

In addition, the balloon catheter transports the balloon to a lesion inside a body lumen. Since the balloon needs to pass through the inside of the bent body lumen while being transported, the balloon is required to have flexibility so as to follow the bent state of the body lumen. However, the technology in the related art, in which a reinforcement member is provided in a wall configuring a balloon, has a problem in that since the reinforcement member is integrally fixed to the balloon and there is no degree of freedom for movement with respect to the wall of the balloon, it is difficult to apply sufficient flexibility to the balloon.

### Summary of Invention

The present invention has been made in consideration of such a problem, and an object thereof is to provide a catheter in which flexibility of a balloon reinforced with a reinforcement member can be improved, and a method of manufacturing the same.

In order to achieve the above-described object, according to the present invention, there is provided a catheter including a balloon which has an inner layer and an outer layer having elastic stretching properties, having tubular shapes, and being able to be inflated and deflated in response to a change of internal pressure; and a tubularly net-shaped reinforcement member which is provided between the inner layer and the outer layer and at least a part of which is not directly fixed to the inner layer and the outer layer. The reinforcement member has a first wire member formed of a high-strength fiber. Both end portions of the reinforcement member in an axial direction are respectively provided with ring-shaped inflation restriction portions welded to the first wire member, and the inflation restriction portions respectively restrict dilation of both the end portions in a circumferential direction. Here, regarding "the reinforcement member", the expression "at least a part of which is not directly fixed to the inner layer and the outer layer" denotes that at least a part of the reinforcement member is not bonded to the inner layer and the outer layer and is not embedded in the inner layer and the outer layer, thereby being able to freely move inside a space formed between the inner layer and the outer layer.

According to the configuration, the reinforcement member having the first wire member formed of a high-strength fiber is disposed between the inner layer and the outer layer of the balloon. Therefore, high-pressure resistance and low compliance properties can be suitably applied to the balloon. Here, the term low compliance properties denotes characteristics in which when the balloon is inflated under high pressure, a balloon diameter is not unlimitedly widened in response to the pressure, and inflation under high pressure can be performed in an appropriate diameter. In addition, the term high-strength fiber denotes a fiber of which tensile break strength is equal to or greater than 2 GPa and elastic modulus is equal to or greater than 50 GPa.

In addition, the reinforcement member has the degree of freedom for moving with respect to the balloon. Therefore, favorable flexibility of the balloon can be maintained. Accordingly, even inside a body lumen which is meandering in a complicated manner, a balloon having high crossability can be realized.

Moreover, when the balloon is inflated, the inflation restriction portions restrict inflation of both the end portions of the reinforcement member in the axial direction. Therefore, the balloon can be inflated so as to have a desired shape inside a body lumen, and a procedure can be effectively performed with respect to a lesion.

In the catheter, the reinforcement member may have a second wire member formed of a fusible material, and the second wire member may be configured with the same material as the inflation restriction portion. In this configuration, the inflation restriction portion is formed of a fusible material and is welded to the first wire member. Therefore, the reinforcement member in which inflation of both the end portions is restricted can be conveniently manufactured.

In the catheter, a tubularly net-shaped body may be formed of the first wire member. The multiple second wire members may be disposed so as to be spaced in the circumferential direction of the tubularly net-shaped body, and the second wire members may individually extend along the tubularly net-shaped body from one end portion to the other end portion of the tubularly net-shaped body and be respectively interlocked with the inflation restriction portions at both the end portions. In this configuration, the tubularly net-shaped body conducting a pressure-resistant function is configured with the first wire member formed of a high-strength fiber. As a configuration independent from the tubularly net-shaped body, the second wire member conducting a welding function is disposed along the tubularly net-shaped body. Therefore, a portion conducting the pressure-resistant function and a portion conducting the welding function are established as the configurations independent from each other. Accordingly, the pressure-resistant function and the welding function can be individually set. Thus, the reinforcement member which has desired pressure resistance and in which inflation of both the end portions is restricted can be simply established.

In the catheter, the first wire member and the second wire member may form the tubularly net-shaped body. In this configuration, as a configuring member of the tubularly net-shaped body, the first wire member and the second wire member are knitted together. Therefore, a base material sleeve is prepared by knitting the first wire member and the second wire member, and the base material sleeve is heated and cut. Accordingly, the reinforcement member in which the inflation restriction portions are formed at both the end portions can be simply manufactured.

In the catheter, a gross-sectional area of a cross-section perpendicular to the axial direction in the first wire member may be greater than a gross-sectional area in the second wire member. According to the configuration, pressure resistance required in the reinforcement member can be suitably ensured.

In addition, according to the present invention, there is provided a catheter manufacturing method of manufacturing a catheter that includes a balloon which has an inner layer and an outer layer having elastic stretching properties, having tubular shapes, and being able to be inflated and deflated in response to a change of internal pressure, and a tubularly net-shaped reinforcement member which is disposed between the inner layer and the outer layer. The catheter manufacturing method includes a base material sleeve preparation step of preparing a base material sleeve including a first wire member formed of a high-strength fiber and a second wire member formed of a fusible material, and a heating and cutting step of heating and cutting multiple spots of the base material sleeve in an axial direction and preparing the reinforcement member in which ring-shaped inflation restriction portions that are formed of fusible materials and are welded to the first wire member are respectively formed at both end portions in the axial direction.

According to the catheter manufacturing method, the base material sleeve is heated and cut. Therefore, when the reinforcement member having a desired length is cut out from the base material sleeve, the fusible material is fused at the heated portion and the ring-shaped inflation restriction portion welded to the first wire member is formed. In contrast, in a case where the reinforcement member is configured with only the first wire member (high-strength fiber), the high-strength fiber is not generally fused and there is little (extremely low) welding properties. Therefore, fibers are unlikely to be welded to each other at both the end portions of the reinforcement member, and both the end portions are not restricted. Thus, according to the catheter manufacturing method of the present invention, the reinforcement member in which inflation of both the end portions is restricted can be conveniently manufactured.

In the catheter manufacturing method, in the base material sleeve preparation step, a state where the multiple second wire members are disposed along the tubularly net-shaped body formed of the first wire member so as to be spaced in a circumferential direction of the tubularly net-shaped body may be established. Accordingly, the pressure-resistant function and the welding function can be individually set. Therefore, the reinforcement member which has desired pressure resistance and in which inflation of both the end portions is restricted can be simply established.

In the catheter manufacturing method, in the base material sleeve preparation step, outer sides of the multiple second wire members disposed along an outer surface of a mandrel may be covered with the tubularly net-shaped body. Accordingly, the multiple second wire members are held between the tubularly net-shaped body and the mandrel. Therefore, the reinforcement member can be efficiently prepared.

In the catheter manufacturing method, in the base material sleeve preparation step, the first wire member and the second wire member may form the tubularly net-shaped base material sleeve. Accordingly, a base material sleeve is prepared by knitting the first wire member and the second wire member, and the base material sleeve is heated and cut. Accordingly, the reinforcement member in which the inflation restriction portions are formed at both the end portions can be simply manufactured.

In the catheter manufacturing method, in the base material sleeve preparation step, the base material sleeve may be formed such that a gross-sectional area of a cross-section perpendicular to the axial direction of the base material sleeve in the first wire member is greater than a gross-sectional area in the second wire member. Accordingly, pressure resistance required of that in the reinforcement member can be suitably ensured.

According to the present invention, in the catheter and the method of manufacturing the same, flexibility of the balloon reinforced with the reinforcement member can be improved.

### Brief Description of Drawings

[Fig. 1] Fig. 1 is a partially-omitted schematic view illustrating a configuration of a catheter, according to an embodiment of the present invention.
[Fig. 2] Fig. 2 is a schematic cross-sectional view of the distal portion of the catheter illustrated in Fig. 1.
[Fig. 3] Fig. 3A is a side view illustrating a reinforcement member when being inflated, and Fig. 3B is a side view illustrating the reinforcement member when being deflated.
[Fig. 4] Fig. 4A is a view describing a step of preparing a base material sleeve, Fig. 4B is a cross-sectional view taken along line IVB-IVB in Fig. 4A, Fig. 4C is a cross-sectional view of a welded portion, and Fig. 4D is a view describing a step of preparing the multiple reinforcement members from the base material sleeve.
[Fig. 5] Fig. 5A is a view describing a step of covering an inner layer tube with the reinforcement member, and Fig. 5B is a view describing a step of covering the inner layer tube and the reinforcement member with an outer layer tube.
[Fig. 6] Fig. 6A is a first view describing a step of joining the inner layer tube and the outer layer tube to each other, and Fig. 6B is a second view describing the step of joining the inner layer tube and the outer layer tube to each other.
[Fig. 7] Fig. 7A is a first view describing a step of joining the distal end of a shaft and the proximal end of a balloon to each other, and Fig. 7B is a second view describing the step of joining the distal end of the shaft and the proximal end of the balloon to each other.
[Fig. 8] Fig. 8A is a first view describing a step of joining an inner tube and the distal end of the balloon to each other, and Fig. 8B is a second view describing the step of joining the inner tube and the distal end of the balloon to each other.
[Fig. 9] Fig. 9A is a first view describing a step of joining a distal tip and the inner tube to each other, and Fig. 9B is a second view describing the step of joining the distal tip and the inner tube to each other.
[Fig. 10] Fig. 10 is a graph illustrating a relationship between pressure and a balloon diameter regarding balloons having forms of fixing the reinforcement member which are different from each other, and a balloon provided with no reinforcement member.
[Fig. 11] Fig. 11 is a side view when the reinforcement member according to a modification example is inflated.

### Description of Embodiment

Hereinafter, a catheter according to the present invention, and a method of manufacturing the same will be described based on a preferable embodiment with reference to the accompanying drawings.

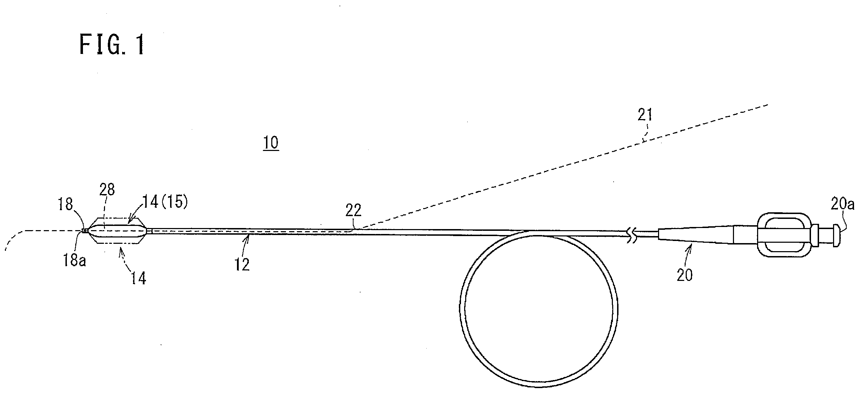

Fig. 1 is a partially-omitted schematic view illustrating a configuration of a catheter 10, according to the embodiment of the present invention. The catheter 10 is a so-called PTCA (percutaneous transluminal coronary angioplasty: percutaneous coronary intervention) inflation catheter for performing treatment in which a long shaft 12 is inserted through a biological organ, for example, the coronary artery, a balloon 14 provided on the distal side thereof is inflated at a stenosed portion (lesion), and the stenosed portion is widened.

The present invention can also be applied to a catheter other than the PTCA dilation catheter, for example, a catheter for improving a lesion formed inside biological organs such as other blood vessels, the bile duct, the trachea, the esophagus, the urethra, and other internal organs.

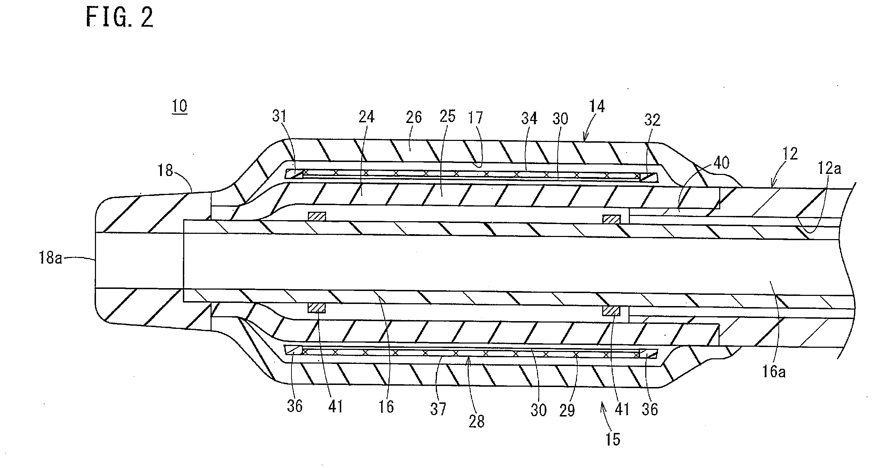

As illustrated in Fig. 1 (and Fig. 2), the catheter 10 includes the long shaft 12 having a small diameter, the balloon 14 joined to the distal end of the shaft 12, a reinforcement member 28 disposed inside a membrane (wall) configuring the balloon 14, an inner tube 16 inserted through the shaft 12 and the balloon 14, a distal tip 18 joined to the distal end of the balloon 14, and a hub 20 provided on the proximal side of the shaft 12.

In Fig. 1, the catheter 10 is configured as a so-called "rapid exchange-type" catheter provided with an opening portion 22 through which a guide wire 21 is guided out, in a middle portion of the shaft 12 in a longitudinal direction. In another embodiment, the catheter 10 may be configured as an "over-the-wire-type" catheter in which a guide wire lumen is formed throughout the overall length of the catheter 10, and the guide wire 21 is guided out from the proximal end of the hub 20.

The shaft 12 is a flexible tube of which both ends in an axial direction are open, and which is long and has a small diameter. The shaft 12 extends from the rear end of the balloon 14 to the distal end of the hub 20. A portion from the distal end to the opening portion 22 configures a double tube which forms a inflation lumen 12a between the shaft 12 and the inner tube 16, and a portion from the opening portion 22 to the hub 20 is a single tube. The shaft 12 forms the inflation lumen 12a through which a inflation fluid for the balloon 14 is supplied.

In the shaft 12, the inflation fluid pressure-fed from a pressure applying apparatus such as an indeflator (not illustrated) connected via a luer taper 20a or the like provided in the hub 20 can be fed to the balloon 14. For example, the inflation fluid is a contrast agent, a physiological salt solution, or a mixture thereof.

The inner tube 16 is a guide wire tube forming a wire lumen 16a through which the guide wire 21 is inserted. The distal end of the inner tube 16 is positioned on the distal side beyond the proximal end of the distal tip 18. The inner tube 16 extends inside the balloon 14 and the shaft 12, and the proximal end thereof is liquid-tightly joined to the opening portion 22 (refer to Fig. 1) formed in an intermediate portion of the shaft 12. Therefore, the guide wire 21 which has inserted through a distal end opening portion 18a serving as an entrance at the distal tip 18 is inserted through the wire lumen 16a of the inner tube 16 from the distal side toward the proximal side and is guided out through the opening portion 22 serving as an exit.

It is favorable to provide a radiopaque marker 41 on the inner tube 16 inside the balloon 14. The radiopaque marker 41 is configured with an X-ray opaque (radiopaque) material (for example, gold, platinum, tungsten, or a mixture thereof). The radiopaque marker 41 is used for visually recognizing the position of the balloon 14 in a living body under an X-ray contrast condition. For example, the radiopaque marker 41 can be configured to have a tubular shape (ring shape). Note that, as in Fig. 2, multiple radiopaque markers 41 may be provided on the inner tube 16 inside the balloon 14 while being spaced from each other in the axial direction. Otherwise, only one radiopaque marker 41 may be provided on the inner tube 16 inside the balloon 14.

It is preferable that the shaft 12 and the inner tube 16 have structures with appropriate flexibility and appropriate rigidity such that an operator can smoothly insert the long catheter 10 into a biological organ such as a blood vessel while grasping and operating the proximal side of the catheter 10. Therefore, for example, it is favorable that the shaft 12 and the inner tube 16 are formed of a polymeric material such as polyolefin (for example, polyethylene, polypropylene, polybutene, an ethylene-propylene copolymer, an ethylene-vinyl acetate copolymer, ionomer, and a mixture of two or more types thereof), polyvinyl chloride, polyamide, a polyamide elastomer, polyurethane, a polyurethane elastomer, polyimide, and a fluorine resin, or a mixture thereof; or a a multi-layer tube including two or more types thereof.

The balloon 14 can be inflated and dilated in response to a change of internal pressure. The distal portion of the balloon 14 is joined to a portion in the vicinity of the distal portion of the inner tube 16, and the proximal portion of the balloon 14 is joined to the distal portion of the shaft 12. The internal space of the balloon 14 communicates with the inflation lumen 12a.

Via the inflation lumen 12a, the inflation fluid can flow into (be guided into) the balloon 14 and the inflation fluid can be discharged from the balloon 14. In response to the inflation fluid guided into the balloon 14, the balloon 14 is inflated. As indicated with the imaginary line in Fig. 1, when the balloon 14 is maximally inflated, a portion between the distal end and the proximal end exhibits a shape which is increased in diameter and has a substantially uniform outer diameter along the axial direction.

The balloon 14 is required to have appropriate flexibility so as to be able to pass through a meandering or bent point of a body lumen. In addition, the balloon 14 is required to have strength to the extent that a lesion can be reliably widened, and the balloon 14 is also required to have high-pressure resistance and low compliance properties. Therefore, in the present embodiment, the balloon 14 has an inner layer 24 and an outer layer 26 having tubular shapes, having elastic stretching properties, and configuring fluid-impermeable balloon walls. The reinforcement member 28 is disposed between the inner layer 24 and the outer layer 26. The balloon 14 and the reinforcement member 28 configure a dilation portion 15 which can be inflated and deflated in a radial direction at the distal portion of the catheter 10.

The inner layer 24 transfer force to the reinforcement member 28 in response to the inflation fluid guided into the balloon 14 (pressurization), and the inner layer 24 expands to the extent of a shape which is restricted along the inflated shape of the reinforcement member 28. The outer layer 26 expands along the inflated shape of the reinforcement member 28 in response to the inflation fluid guided into the balloon 14 (pressurization), and the outer layer 26 contracts to the extent of the initial shape in response to the inflation fluid discharged from the inside of the balloon 14 (decompression) in order to restore the original shape (position) of the reinforcement member 28 before being inflated. Therefore, it is preferable that the outer layer 26 is formed of a base material having a high stretching recovery rate.

The inner layer 24 and the outer layer 26 are joined to each other at the distal portions and the proximal portions, for example, through welding or bonding. An annularly sealed accommodation chamber 17 accommodating the reinforcement member 28 is formed between the inner layer 24 and the outer layer 26.

Examples of the configuring materials of the inner layer 24 and the outer layer 26 include various types of rubber material such as natural rubber, butyl rubber, isoprene rubber, butadiene rubber, styrene-butadiene rubber, and silicone rubber; various types of thermoplastic elastomer such as a polyurethane-based elastomer, a polyester-based elastomer, a polyamide-based elastomer, an olefin-based elastomer, and a styrene-based elastomer; mixtures thereof; and the like. The configuring material of the inner layer 24 and the configuring material of the outer layer 26 may be the same as each other or may be different from each other.

The reinforcement member 28 is a tubularly net-shaped member which is disposed between the inner layer 24 and the outer layer 26 such that at least a part thereof is movable with respect to the balloon 14, and the reinforcement member 28 functions to enhance pressure resistance of the balloon 14.

The reinforcement member 28 has both end portions (first end portion 31 and second end portion 32) in the axial direction, and an intermediate portion 34 configuring the middle between the first end portion 31 and the second end portion 32. In the reinforcement member 28, at least one of the first end portion 31 or the second end portion 32, and the intermediate portion 34 are not directly fixed to the inner layer 24 and the outer layer 26. Accordingly, movement with respect to the inner layer 24 and the outer layer 26 in the axial direction and a circumferential direction is allowed.

The inner layer 24 and the outer layer 26 may be fixedly attached (for example, welded or bonded) via a gap (mesh) between first threads 29 forming the reinforcement member 28. Accordingly, while the reinforcement member 28 is allowed to move with respect to the inner layer 24 and the outer layer 26 to a certain extent, the moving range of the reinforcement member 28 can be restricted.

In a case of the present embodiment, the other one of the first end portion 31 and the second end portion 32 is not also directly fixed to the inner layer 24 and the outer layer 26. Accordingly, movement with respect to the inner layer 24 and the outer layer 26 in the axial direction is allowed. In other words, in the present embodiment, the reinforcement member 28 is not fixed to any one of the inner layer 24 and the outer layer 26. Therefore, the reinforcement member 28 can freely move in the circumferential direction and the axial direction within a range restricted by the inner layer 24 and the outer layer 26 (within a range of the accommodation chamber 17).

Note that, only one of the first end portion 31 or the second end portion 32 may be fixed to the inner layer 24 or the outer layer 26. In this case, fixing means is not limited to any particular means and suitable fixing means such as welding and bonding may be employed.

The reinforcement member 28 has the first thread 29 (first wire member) formed of a high-strength fiber, and a second thread 30 (second wire member) formed of a fusible material. In addition, both the end portions of the reinforcement member 28 in the axial direction are respectively provided with ring-shaped inflation restriction portions 36 which are formed of fusible materials and are welded to the first thread 29. The inflation restriction portions 36 restrict inflation of both the end portions (first end portion 31 and second end portion 32) in the circumferential direction.

In the present embodiment, specifically, one or more first threads 29 form a tubularly net-shaped body 37. The tubularly net-shaped body 37 is formed so as to be a tubularly net-shaped body by knitting (weaving) one or more first threads 29, and the tubularly net-shaped body 37 has stretching properties in at least the circumferential direction (and the radial direction).

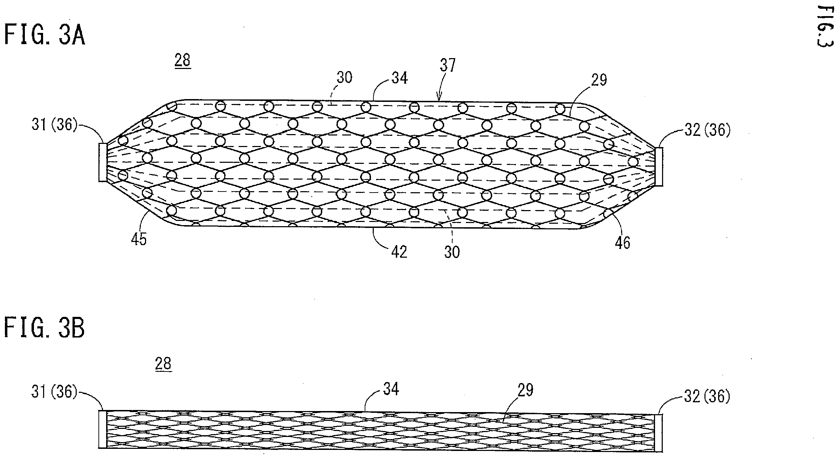

The method of forming the tubularly net-shaped body 37 is not limited to any particular form. Examples of the method include tube-knitting and braiding. In a case of tube-knitting, in the tubularly net-shaped body 37, the first threads 29 extending in the circumferential direction in a waved manner are arranged in the axial direction, and the waved first threads 29 adjacent to each other in the axial direction are interlaced with each other (refer to Fig. 3A). In a case of braiding, in the tubularly net-shaped body 37, one or more first threads 29 extending in a first spiral direction and one or more first threads 29 extending in a second spiral direction are woven so as to intersect each other, thereby forming a tubular shape.

The high-strength fiber denotes a fiber of which tensile break strength is equal to or greater than 2 GPa and elastic modulus is equal to or greater than 50 GPa. The high-strength fiber is also referred to as a super fiber. It is preferable that the first thread 29 is a twisted thread formed of the high-strength fiber. Examples of the high-strength fiber include an aramid fiber, a carbon fiber, a polyarylate fiber, a PBO fiber, ultra-high molecular weight polyethylene, and an LCP fiber. Note that, generally, since the high-strength fiber is not fused even when being heated, there is no welding properties or extremely low welding properties with respect to other members.

For example, the diameter of the first thread 29 may range approximately from 5 to 100 µm. In a case where the twisted thread formed of the high-strength fibers is used as the first thread 29, for example, a single fiber diameter of the high-strength fiber may range approximately from 5 to 30 µm. As the high-strength fiber, for example, a fiber having a single fiber diameter of 12 µm can be used. However, a thinner fiber may be used, and a thicker fiber may be used. In a case of a thicker fiber, it is favorable to employ a loosely twisted thread so as to be in an unraveled state when tensile force is not applied to the twisted thread.

In addition, in the reinforcement member 28, the multiple second threads 30 formed of fusible materials are disposed so as to be spaced in the circumferential direction of the tubularly net-shaped body 37. Here, the fusible material denotes a material which can be softened and fused by being heated to a predetermined temperature or higher, and the fusible material has welding properties with respect to other members.

The second threads 30 individually extend along the tubularly net-shaped body 37 from one end portion to the other end portion of the tubularly net-shaped body 37 and are respectively interlocked with the inflation restriction portions 36 at both the end portions. In addition, in a region between the inflation restriction portions 36, the second thread 30 is not bonded and is not welded to the tubularly net-shaped body 37 (first thread 29). Therefore, the second thread 30 is not fixed.

The inflation restriction portion 36 is a portion formed during the process of manufacturing the reinforcement member 28, in which the multiple second threads 30 arranged in the circumferential direction are fused, the fused material flows in the circumferential direction and is solidified thereafter. In order to establish such a inflation restriction portion 36, the number of the second threads 30, disposition spaces in the circumferential direction, the thickness, and/or the like are set such that the fused material is connected to form a ring shape in accordance with fusion and flowing when the second threads 30 are heated and the fused material has strength (rigidity) to the extent that inflation of both the end portions of the reinforcement member 28 can be reliably restricted.

Examples of the configuring material (fusible material) of the second thread 30 include polyvinyl chloride, polyethylene, polypropylene, annular polyolefin, polystyrene, poly-(4-methylpentene-1), polycarbonate, an acrylic resin, an acrylonitrile-butadiene-styrene copolymer, polyester such as polyethylene terephthalate and polyethylene naphthalate, a butadiene-styrene copolymer, a polyamide (for example, nylon 6, nylon 6.6, nylon 6.10, and nylon 12), and a polyamide elastomer.

In the present embodiment, the second threads 30 are disposed on the inner surface side of the tubularly net-shaped body 37. Note that, the second threads 30 may be disposed on the outer surface side of the tubularly net-shaped body 37.

As in Fig. 2, each of the second threads 30 may be disposed so as to extend along the axial direction of the reinforcement member 28 when being deflated. Otherwise, each of the second threads 30 may be disposed so as to extend in a direction tilting with respect to the axial direction of the reinforcement member 28 (that is, in a spiral manner).

As illustrated in Fig. 2, the distal portion of the inner layer 24 is joined to the inner tube 16. In addition, the proximal portion of the inner layer 24 is joined to the distal portion (thin portion 40) of the shaft 12, and the outermost distal portion of the shaft 12 is positioned on the distal side beyond the innermost proximal surface of the inner layer 24, on the inner side of the inner layer 24. Therefore, a stretchable region (hereinafter, will be referred to as "stretchable region 25 of the inner layer 24") in the inner layer 24 during deformation of inflation and deflation of the balloon 14 is a portion between a joint spot of the inner layer 24 and the inner tube 16, and the outermost distal portion of the shaft 12.

The innermost proximal portion of the reinforcement member 28 is positioned on the proximal side beyond the innermost proximal portion of the stretchable region 25 in the inner layer 24. Note that, as illustrated in Fig. 2, the second end portion 32 of the reinforcement member 28 may be disposed on the proximal side beyond the outermost distal portion of the shaft 12 in the accommodation chamber 17 formed between the inner layer 24 and the outer layer 26 of the balloon 14. Accordingly, when the balloon 14 is dilated, the second end portion 32 of the reinforcement member 28 is less affected by inflation of the balloon 14 and contributes to restriction of the maximally inflated diameter of the balloon 14 performed by the reinforcement member 28.

Fig. 3A is a side view illustrating the reinforcement member 28 when being inflated, and Fig. 3B is a side view illustrating the reinforcement member 28 when being deflated. As illustrated in Fig. 3A, when the reinforcement member 28 is inflated in the circumferential direction, the first threads 29 are in a tensed state, and the outer diameter thereof is not increased to a certain extent or greater. In this case, since inflation of the first end portion 31 and the second end portion 32 is restricted, the shape of the reinforcement member 28 (intermediate portion 34) when being inflated includes a straight portion 42 having a substantially uniform outer diameter, and outer-diameter varying portions (tapered portions) 45 and 46 which are respectively positioned on both sides of the straight portion 42 and are decreased in diameter outward in the axial direction.

Note that, in a case where the balloon 14 is produced by using the reinforcement member 28 illustrated in Figs. 3A and 3B, the balloon 14 when being dinflated includes a straight portion having a substantially uniform outer shape due to the reinforcement member 28, and outer-diameter varying portions (tapered portions) which are respectively positioned on both sides of the straight portion and are decreased in diameter outward in the axial direction. In such a case, the radiopaque marker 41 is disposed on the inner tube 16 such that the position of the straight portion of the balloon 14 can be perceived. Accordingly, since an operator can visually recognize the position having the maximally inflated diameter in the balloon 14 under an X-ray contrast condition, positioning between the region of the maximally inflated diameter in the balloon 14, and the lesion can be easily performed.

In a case of the reinforcement member 28 having the tubularly net-shaped body 37 formed through the knitting method in which the waved first threads 29 adjacent to each other in the axial direction are interlaced with each other, when the reinforcement member 28 is compressed in the circumferential direction as illustrated in Fig. 3B, the first threads 29 are folded and the reinforcement member 28 (tubularly net-shaped body 37) is decreased in diameter. In addition, when the reinforcement member 28 is compressed in the axial direction, the first threads 29 of the meshes are misaligned and the first threads 29 adjacent to each other in the axial direction can overlap each other. Moreover, the reinforcement member 28 can be bent in accordance with rotations of the interlaced portion between the first threads 29 adjacent to each other in the axial direction. Therefore, such a reinforcement member 28 has excellent flexibility with respect to bending.

In Figs. 1 and 2, the distal tip 18 provided on the distal side of the balloon 14 is a portion which flexibly advances through a curved portion, an irregular portion, and the like inside a biological organ, as the outermost distal end of the catheter 10, penetrates a lesion (stenosed portion), and leads the catheter 10 to be smoothly inserted through. The distal tip 18 is a short tube having an inner diameter substantially the same as the inner diameter of the inner tube 16.

The distal tip 18 is fitted to the distal portion of the inner tube 16 from the outside so as to be liquid-tightly joined to the distal portion of the inner tube 16. The distal tip 18 protrudes toward the distal side beyond the distal end opening portion 18a of the wire lumen 16a, and the proximal surface thereof is joined to the distal surface of the balloon 14. The distal end opening portion 18a of the distal tip 18 communicates with the wire lumen 16a of the inner tube 16 and serves as the entrance of the guide wire 21.

The material and the shape of the distal tip 18 are suitably set such that the distal tip 18 is configured to be more flexible than at least the shaft 12 and the inner tube 16. Note that, the distal tip 18 may be omitted. In such a case, it is favorable to employ a configuration in which the outermost distal end position of the inner tube 16 and the outermost distal end position of the balloon 14 coincide with each other, or a configuration in which the outermost distal end position of the inner tube 16 slightly protrudes beyond the outermost distal end position of the balloon 14.

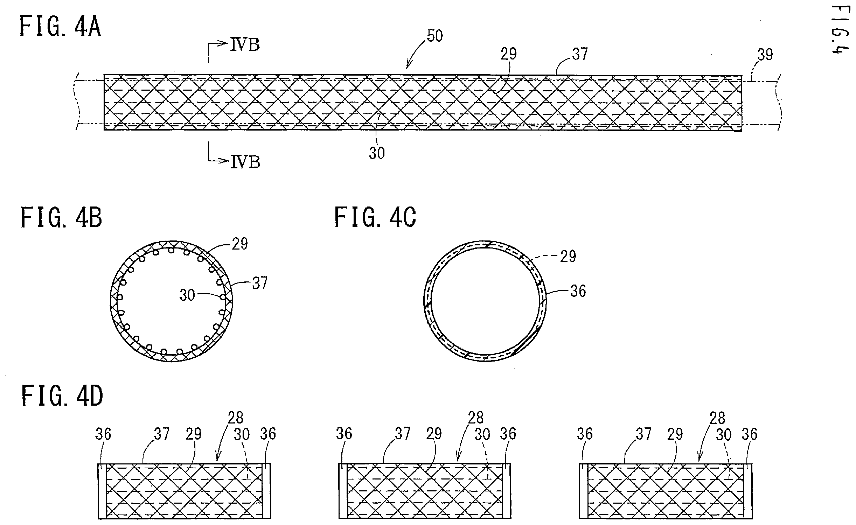

Subsequently, an example of a method of manufacturing the catheter 10 (mainly, a step of manufacturing the inflation portion 15 and peripheral portions thereof) will be described. Note that, the present invention is not limited to the exemplified manufacturing method. In Figs. 4A to 9B, the tubularly net-shaped body 37 is schematically illustrated, and the tubularly net-shaped body 37 is not limited to any particular knitting method.

Figs. 4A to 4D are views describing the step of manufacturing the reinforcement member 28. As in Fig. 4A, first, a step of preparing a tubularly net-shaped base material sleeve 50 which becomes the base material of the reinforcement member 28 (base material sleeve preparation step) is executed. The base material sleeve 50 has a length equal to or greater than those of multiple reinforcement members 28.

In the base material sleeve preparation step, specifically, a state where the multiple second threads 30 are disposed along the tubularly net-shaped body 37 formed of the first thread 29 so as to be spaced in the circumferential direction of the tubularly net-shaped body 37 is established (also refer to Fig. 4B which is a cross-sectional view taken along line IVB-IVB in Fig. 4A). In this case, as in Fig. 4A, it is favorable that the multiple second threads 30 are disposed so as to be spaced in the circumferential direction along the outer surface of a mandrel 39 and the outer sides of the disposed multiple second threads 30 are covered with the tubularly net-shaped body 37. Accordingly, the multiple second threads 30 are held between the tubularly net-shaped body 37 and the mandrel 39. Therefore, there is no need to provide a separate mechanism for holding the second threads 30, and the second threads 30 can be appropriately disposed in an efficient manner.

Subsequently, multiple spots of the base material sleeve 50 are heated and cut in the axial direction, thereby executing a step of preparing one or more reinforcement members 28 (multiple reinforcement members 28 in Fig. 4D) in each of which the ring-shaped inflation restriction portions 36 that are formed of fusible materials and are welded to the first thread 29 are respectively formed at both end portions in the axial direction (heating and cutting step) as in Fig. 4D,.

In this case, the base material sleeve 50 is heated and cut. Accordingly, when the reinforcement member 28 having a desired length is cut out from the base material sleeve 50, the second threads 30 are fused at the heated portion, and the fused material flows and spreads in the circumferential direction of the base material sleeve 50. As a result thereof, a change occurs in the structure at the heated portion as in Figs. 4B and Fig. 4C, and the ring-shaped inflation restriction portions 36 welded to the first thread 29 are formed.

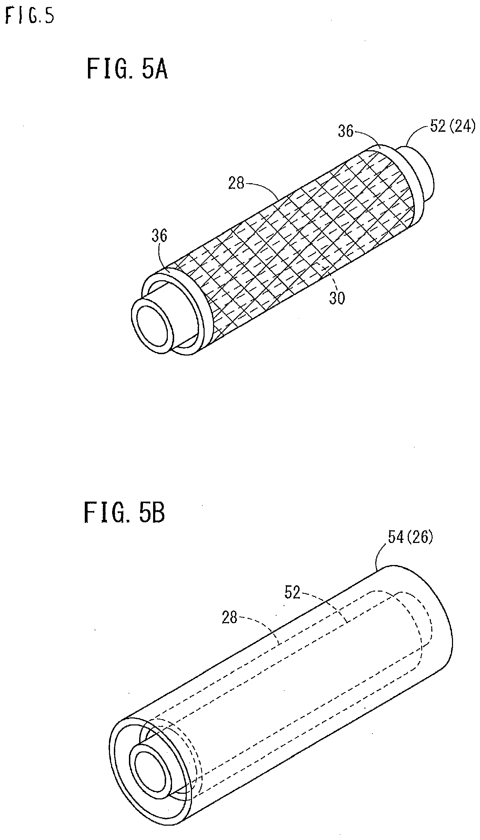

Subsequently, as in Fig. 5A, a step of covering an inner layer tube 52 which is the base material of the inner layer 24, with the reinforcement member 28 (first covering step) is executed. In this case, both end portions of the inner layer tube 52 respectively protrude beyond openings at both ends of the reinforcement member 28.

Subsequently, as in Fig. 5B, a step of covering the inner layer tube 52 and the reinforcement member 28 (reinforcement member 28 in a state where the inner layer tube 52 is inserted into the inner side) with an outer layer tube 54 which is the base material of the outer layer 26 (second covering step) is executed. In this case, the inner layer tube 52 and the reinforcement member 28 are covered with the outer layer tube 54 such that the overall length of the reinforcement member 28 is housed inside the outer layer tube 54 (both end portions of the outer layer 26 protrude in the axial direction beyond both the end portions of the reinforcement member 28).

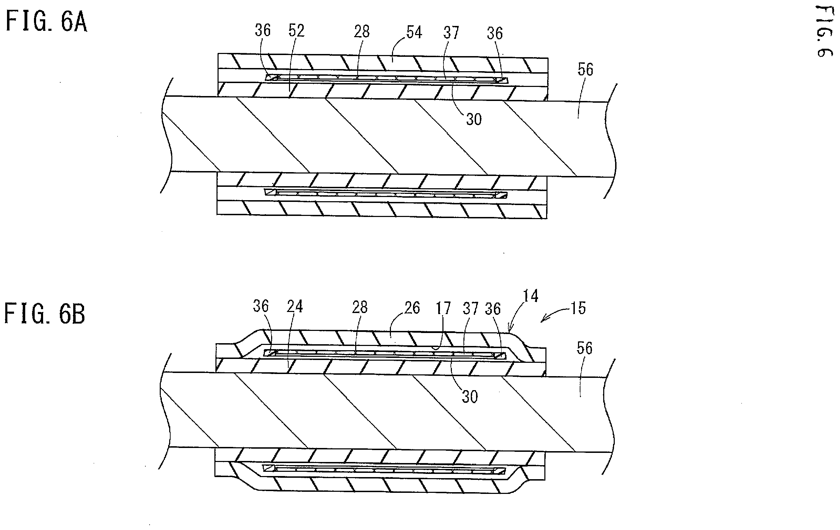

Subsequently, a step of joining the inner layer tube 52 and the outer layer tube 54 (step of joining inner and outer layers) is executed. Specifically, first, as in Fig. 6A, a mandrel 56 (core bar) is inserted into the inner layer tube 52 (assembly of the inner layer tube 52, the outer layer tube 54, and the reinforcement member 28). Subsequently, as in Fig. 6B, the inner layer tube 52 and the outer layer tube 54 are welded so as to be joined to each other at one end portions and the other end portions. Accordingly, the annularly sealed accommodation chamber 17 is formed between the inner layer 24 and the outer layer 26, thereby obtaining the inflation portion 15 in a state where the reinforcement member 28 is disposed inside the accommodation chamber 17. After the step of joining inner and outer layers, the mandrel 56 is removed.

In this case, in the present embodiment, the reinforcement member 28 is merely disposed inside the accommodation chamber 17 and is not joined to other members through welding, bonding, or the like. Therefore, the reinforcement member 28 is not fixed to any portion of the balloon 14 (inner layer 24 and outer layer 26).

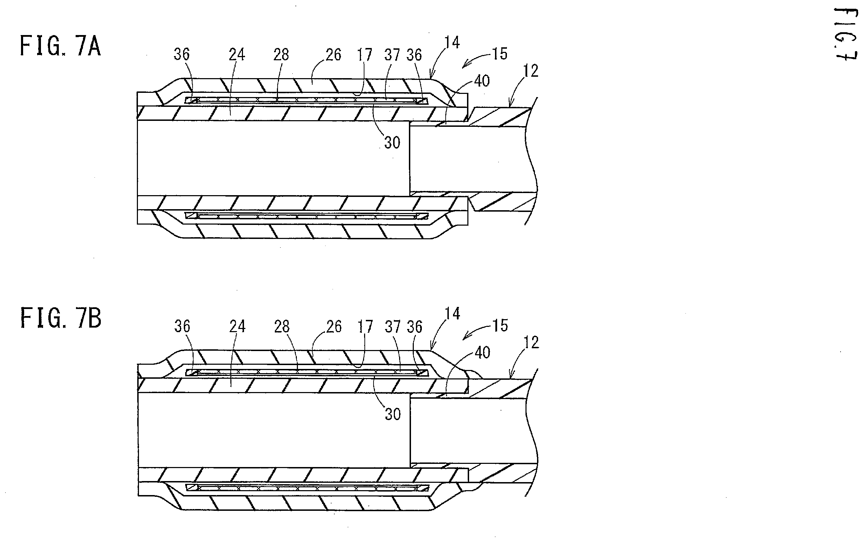

Subsequently, a step of joining the balloon 14 (inflation portion 15) and the shaft 12 to each other (step of joining a balloon and a shaft) is executed (Figs. 7A and 7B). Specifically, the thin portion 40 is formed at the distal portion of the shaft 12. In this case, for example, the distal portion of the shaft 12 is drawn down (the mandrel is inserted into a hollow portion of the shaft 12, and the distal portion of the shaft 12 is pressedly input into a mold which includes a hole having a diameter smaller than that of the shaft 12), and thus, the distal portion can have a small diameter. Subsequently, as in Fig. 7A, the thin portion 40 of the shaft 12 is inserted into the proximal side of the balloon 14. Subsequently, as in Fig. 7B, the proximal portion of the balloon 14 and the distal portion (thin portion 40) of the shaft 12 are welded so as to be joined to each other.

Subsequently, even though the step is not illustrated, the radiopaque marker 41 is attached to the inner tube 16. Specifically, the tubular radiopaque marker 41 having an inner diameter slightly greater than the inner tube 16 is caused to pass through the outer side of the inner tube 16, and the mandrel is inserted into the inner tube 16. Thereafter, the entire circumference of the radiopaque marker 41 is beaten (swaging step). The radiopaque marker 41 is decreased in diameter and is engaged with the inner tube 16. In this manner, the radiopaque marker 41 is fixed to the inner tube 16.

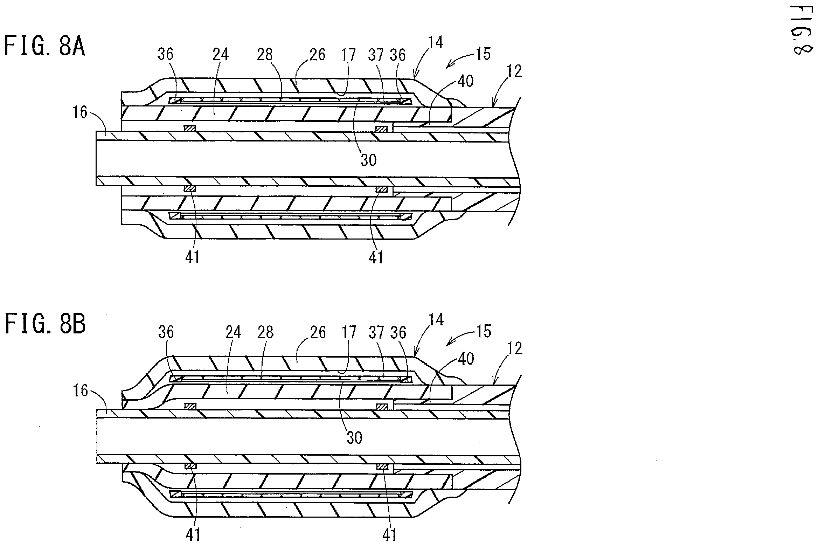

Subsequently, a step of joining the balloon 14 and the inner tube 16 to each other (step of joining a balloon and an inner tube) is executed (Figs. 8A and 8B). Specifically, as in Fig. 8A, the inner tube 16 is inserted into the balloon 14 and the shaft 12. Subsequently, as in Fig. 8B, the distal portion of the balloon 14 and the inner tube 16 are welded so as to be joined to each other.

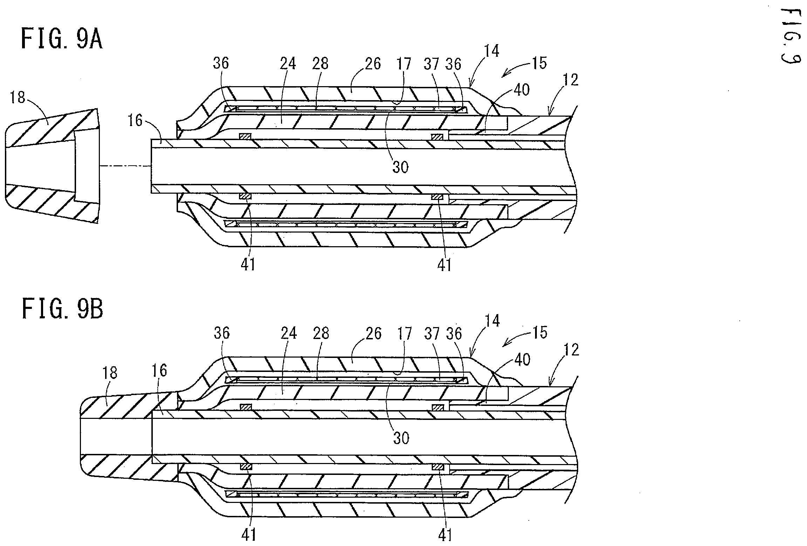

Subsequently, a step of joining the distal tip 18 and the inner tube 16 to each other (step of joining a distal tip and an inner tube) is executed (Figs. 9A and 9B). Specifically, first, the distal portion of the inner tube 16 is cut, and the length is adjusted (Fig. 9A). Subsequently, the proximal portion of the distal tip 18 is fitted to the distal portion of the inner tube 16 from the outside, and the proximal portion of the distal tip 18 and the distal portion of the inner tube 16 are welded so as to be joined to each other (Fig. 9B).

Note that, a step of joining the proximal end of the shaft 12 and the distal portion of the hub 20 to each other (step of joining a shaft and a hub) can be executed at an arbitrary time. For example, the step of joining a shaft and a hub may be executed before the step of joining a balloon and a shaft, may be executed after the step of joining a distal tip and an inner tube, or may be executed between the step of joining a balloon and a shaft and the step of joining a distal tip and an inner tube.

In the above-described manufacturing method, as the means for joining members to each other, welding is exemplified. However, instead of welding, other types of joint means such as bonding may be applied.

The catheter 10 according to the present embodiment is basically configured as described above. Hereinafter, operations and effects thereof will be described.

For example, treatment using the catheter 10 is performed as follows. First, a form of a lesion (stenosed portion) occurred inside a blood vessel is specified through an intravascular contrast method or an intravascular ultrasound diagnosis method. Subsequently, for example, the guide wire 21 is percutaneously guided into a blood vessel in advance through a Seldinger' s method, and the guide wire 21 is inserted through the wire lumen 16a of the inner tube 16 from the distal end opening portion 18a of the distal tip 18. While the guide wire 21 is guided out through the opening portion 22, the catheter 10 is inserted into a blood vessel. Under a radioscopic condition, the guide wire 21 is caused to advance toward a target lesion. The guide wire 21 is caused to pass through the lesion and to indwell, and the catheter 10 is caused to advance along the guide wire 21.

When the distal tip 18 of the catheter 10 passes through the lesion, the balloon 14 is positioned at the lesion. When the inflation fluid (for example, contrast agent) is pressure-fed into the inflation lumen 12a from the hub 20 side, the balloon 14 is inflated and the lesion is widened. Accordingly, treatment of the lesion can be performed. Subsequently, the dilation fluid is suctioned from the inside of the balloon 14 to the hub 20 side through the inflation lumen 12a, and the balloon 14 is deflated again. In a case where an additional lesion required to be treated is present at a different part inside a body lumen, the balloon 14 is delivered to the additional lesion, the balloon 14 is dinflated and deflated in a similar manner as described above. When the procedure for all of the lesion in a treatment object is completed, the catheter 10 is removed from the body.

In this case, in the catheter 10 according to the present embodiment as described above, since the reinforcement member 28 having the first threads 29 formed of the high-strength fibers is disposed between the inner layer 24 and the outer layer 26 of the balloon 14, high-pressure resistance and low compliance properties can be suitably applied to the balloon 14. In addition, since the reinforcement member 28 has the degree of freedom for moving with respect to the balloon 14, favorable flexibility of the balloon 14 can be maintained. Accordingly, it is possible to realize the balloon 14 having high crossability even inside a complicatedly meandering body lumen.

Particularly, in a case of the present embodiment, in the reinforcement member 28, at least one of the first end portion 31 or the second end portion 32, and the intermediate portion 34 are not directly fixed to the balloon 14. In other words, substantially the entirety of the reinforcement member 28 has the degree of freedom for moving in the axial direction and the circumferential direction with respect to the balloon 14. Therefore, favorable flexibility of the balloon 14 can be maintained. Accordingly, it is possible to realize the balloon 14 having high crossability inside a body lumen.

Moreover, in a case of the present embodiment, not only one of the first end portion 31 or the second end portion 32 but also the other one thereof is not directly fixed to any one of the inner layer 24 and the outer layer 26. According to the configuration, the reinforcement member 28 is not fixed to any portion in the balloon 14. Therefore, the degree of freedom for moving the reinforcement member 28 with respect to the balloon 14 can be further improved, and flexibility can be improved. In accordance therewith, crossability inside a body lumen can be further improved.

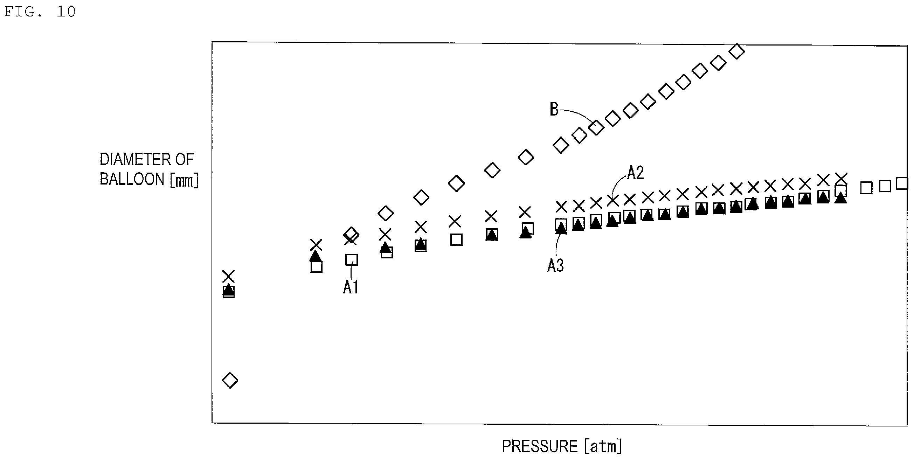

Here, Fig. 10 is a graph illustrating a relationship between pressure and a balloon diameter regarding balloons A1 to A3 which are provided with the reinforcement member 28 and in which forms of fixing the reinforcement member 28 are different from each other, and a balloon B provided with no reinforcement member 28. In Fig. 10, in the balloon A1, both the end portions of the reinforcement member 28 in the axial direction are fixed to the inner layer 24. In the balloon A2, only one end portion of the reinforcement member 28 in the axial direction is fixed to the inner layer 24. In the balloon A3, the reinforcement member 28 is not fixed to any place.

According to Fig. 10, it is found that in the balloons A1 to A3 provided with the reinforcement member 28, compared to the balloon B provided with no reinforcement member 28, the increases of the balloon diameters with respect to the increases of the pressures are gentle, while having high pressure resistance and low compliance properties. Meanwhile, in the balloons A1 to A3 provided with the reinforcement member 28, no meaningful difference based on the forms of fixing the reinforcement member 28 is recognized. Therefore, it is understood that a balloon having high-pressure resistance and low compliance properties can be realized by providing the reinforcement member 28 between the inner layer 24 and the outer layer 26, regardless of whether or not the reinforcement member 28 is fixed. Therefore, from the viewpoint of maintaining favorable flexibility of the balloon 14 and improving crossability of the catheter 10 inside a body lumen, it is favorable that at least one of both the end portions of the reinforcement member 28, and the intermediate portion 34 are not fixed to the inner layer 24 and the outer layer 26 of the balloon 14.

In addition, according to the catheter 10, when the balloon 14 is inflated, the inflation restriction portions 36 restrict inflation of both the end portions of the reinforcement member 28 in the axial direction. Therefore, inside a body lumen, the balloon 14 can be inflated so as to have a desired shape, and a procedure can be effectively performed with respect to a lesion. Besides, the inflation restriction portion 36 is formed of a fusible material and is welded to the first thread 29. Therefore, the reinforcement member 28 in which inflation of both the end portions is restricted can be conveniently manufactured.

In a case of the present embodiment, the tubularly net-shaped body 37 conducting a pressure-resistant function is configured with the first thread 29 formed of a high-strength fiber. As a configuration independent from the tubularly net-shaped body 37, the second thread 30 conducting a welding function is disposed along the tubularly net-shaped body 37. Therefore, a portion conducting the pressure-resistant function and a portion conducting the welding function are established as the configurations independent from each other. Accordingly, the pressure-resistant function and the welding function can be individually set. Thus, the reinforcement member 28 which has desired pressure resistance and in which inflation of both the end portions is restricted can be simply established.

1 In addition, in a case of the present embodiment, the balloon 14 is inflated and deflated while entailing elastic stretching and is a zero folding-type balloon which is not folded when being in a deflated state. Accordingly, the balloon can easily restore the original outer diameter when being deflated again after inflation. Therefore, in a case where multiple lesion occurred in locations different from each other inside a body lumen are treated with the same balloon 14, the outer diameter after being deflated again is restrained from becoming greater than the initial outer diameter. Therefore, even after the balloon 14 is deflated again, favorable crossability inside a body lumen can be maintained.

Furthermore, the balloon 14 having elastic stretching properties can be prepared without performing blow molding. Therefore, the catheter 10 can be conveniently manufactured. In other words, in a case of a balloon which is configured with a non-stretchable material, the balloon is required to be molded so as to have a desired shape by executing blow molding after manufacturing the base material of the balloon. Moreover, in order to cause the balloon to be in a deflated state, there is a need to execute a wrapping step in which the balloon is folded (one or more outer circumferential portions of the balloon are folded in the circumferential direction in an overlapping manner). In contrast, in a case of the balloon 14 of the present embodiment, as it is clear from the above-described manufacturing method, blow molding is not necessary and the wrapping step thereafter is also not necessary. Therefore, it is possible to reduce the number of steps and to lower the manufacturing cost.

In addition, in a case of the present embodiment, in the first end portion 31 and the second end portion 32 of the reinforcement member 28, inflation in the circumferential direction and the radial direction is restricted by the inflation restriction portion 36 (refer to Fig. 2). According to the configuration, in the reinforcement member 28, the maximally inflated diameter of the intermediate portion 34 positioned between the first end portion 31 and the second end portion 32 can be effectively restricted. Therefore, the function as the reinforcement member 28 can be suitably conducted.

In a case of the present embodiment, the reinforcement member 28 is formed by tubularly knitting one or more first threads 29, and the waved first threads 29 adjacent to each other in the axial direction are interlaced with each other (refer to Fig. 3A). According to the configuration, when the reinforcement member 28 is compressed in the circumferential direction, the first threads 29 are folded in the circumferential direction, and when the reinforcement member 28 is compressed in the axial direction, the first threads 29 of the meshes are misaligned in the axial direction. Therefore, the reinforcement member 28 can be flexibly bent.

In addition, in the reinforcement member 28 in which the meandering first threads 29 adjacent to each other in the axial direction are interlaced with each other, the interlaced portion of the first threads 29 configures an interlock portion. In the interlock portion, the first threads 29 are not bonded to each other, and the first threads 29 are formed so as to be movable with respect to each other. According to the configuration, the reinforcement member 28 can be bent in accordance with rotations of the interlaced portion of the first threads 29. Therefore, flexibility of the balloon 14 can be further enhanced.

In addition, according to the catheter manufacturing method of the present embodiment, the base material sleeve 50 is heated and cut. Therefore, when the reinforcement member 28 having a desired length is cut out from the base material sleeve 50, a fusible material is fused at the heated portion and the ring-shaped inflation restriction portion 36 welded to the first thread 29 is formed. In contrast, in a case of a reinforcement member (not illustrated) which is configured with only the first threads 29 (high-strength fiber), the high-strength fiber is not generally fused and there is little (extremely low) welding properties. Therefore, fibers are unlikely to be welded to each other at both the end portions of the reinforcement member, and both the end portions are not restricted. Thus, according to the catheter manufacturing method of the present embodiment, the reinforcement member 28 in which inflation of both the end portions is restricted can be conveniently manufactured.

In addition, as in the present embodiment, in the base material sleeve preparation step, when the outer sides of the multiple second threads 30 disposed along the outer surface of the mandrel 39 is covered with the tubularly net-shaped body 37, the multiple second wire members are held between the tubularly net-shaped body 37 and the mandrel 39. Thus, the reinforcement member 28 can be efficiently prepared.

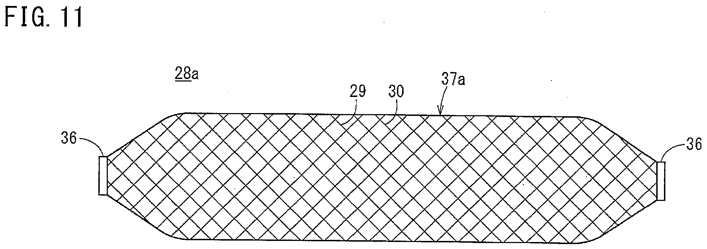

In the catheter 10 described above, in place of the reinforcement member 28, a reinforcement member 28a illustrated in Fig. 11 may be applied. In the reinforcement member 28a, one or more first threads 29 formed of high-strength fibers, and one or more second threads 30 formed of fusible materials form a tubularly net-shaped body 37a. In this case, the method of forming the tubularly net-shaped body 37a is not particularly limited, and the method can be performed through tube-knitting, braiding, and the like as described above.

In this configuration, as the configuring member of the tubularly net-shaped body 37a, the first thread 29 and the second thread 30 are knitted together. Therefore, in the base material sleeve preparation step regarding the manufacture of the reinforcement member 28a, a base material sleeve is prepared by knitting the first thread 29 and the second thread 30, and one or more spots of the base material sleeve in the axial direction are heated and cut. Accordingly, the reinforcement member 28a in which the inflation restriction portions 36 are formed at both the end portions can be simply manufactured.

Particularly, when the gross-sectional area of the cross-section perpendicular to the axial direction of the reinforcement member 28a in the first thread 29 is greater than the gross-sectional area of that in the second thread 30, pressure resistance required in the reinforcement member 28a can be suitably ensured. In this case, in the base material sleeve preparation step regarding the manufacture of the reinforcement member 28a, the base material sleeve is formed such that the gross-sectional area of the cross-section perpendicular to the axial direction of the base material sleeve in the first thread 29 is greater than the gross-sectional area of that in the second thread 30.

For example, the gross-sectional area in the first thread 29 may be greater than the gross-sectional area in the second thread 30 by increasing the number of the first threads 29 configuring the base material sleeve so as to exceed the number of the second threads 30, or increasing the thickness of the first thread 29 configuring the base material sleeve so as to exceed the thickness of the second thread 30. Otherwise, the gross-sectional area in the first thread 29 may be greater than the gross-sectional area in the second thread 30 by adjusting the number and the thickness of the first threads 29 and the second threads 30.

In the description above, the present invention has been described based on the preferable embodiment. However, the present invention is not limited to the embodiment described above, and it is not necessary to mention that various modifications and changes can be made without departing from the scope and the gist of the present invention.

## Claims

1. A catheter (10) comprising:
a balloon (14) which has an inner layer (24) and an outer layer (26) having elastic stretching properties, having tubular shapes, and being able to be inflated and deflated in response to a change of internal pressure; and
a tubularly net-shaped reinforcement member (28, 28a) which is provided between the inner layer (24) and the outer layer (26) and at least a part of which is not directly fixed to the inner layer (24) and the outer layer (26),
wherein the reinforcement member (28, 28a) has a first wire member (29) formed of a high-strength fiber, and
wherein both end portions of the reinforcement member (28, 28a) in an axial direction are respectively provided with ring-shaped inflation restriction portions (36) welded to the first wire member (29), and the inflation restriction portions (36) respectively restrict inflation of both the end portions in a circumferential direction.

2. The catheter (10) according to Claim 1,
wherein the reinforcement member (28, 28a) has a second wire member (30) formed of a fusible material, and
wherein the second wire member (30) is configured with the same material as the inflation restriction portion (36).

3. The catheter (10) according to Claim 2,
wherein a tubularly net-shaped body (37) is formed of the first wire member (29), and
wherein the multiple second wire members (30) are disposed so as to be spaced in the circumferential direction of the tubularly net-shaped body (37), and the second wire members (30) individually extend along the tubularly net-shaped body (37) from one end portion to the other end portion of the tubularly net-shaped body (37) and are respectively interlocked with the inflation restriction portions (36) at both the end portions.

4. The catheter (10) according to Claim 2,
wherein the first wire member (29) and the second wire member (30) form a tubularly net-shaped body (37a).

5. The catheter (10) according to Claim 4,
wherein a gross-sectional area of a cross section perpendicular to the axial direction in the first wire member (29) is greater than a gross-sectional area in the second wire member (30).

6. A catheter manufacturing method of manufacturing a catheter (10) that includes a balloon (14) which has an inner layer (24) and an outer layer (26) having elastic stretching properties, having tubular shapes, and being able to be inflated and deflated in response to a change of internal pressure, and a tubularly net-shaped reinforcement member (28, 28a) which is disposed between the inner layer (24) and the outer layer (26), the catheter manufacturing method comprising:
a base material sleeve preparation step of preparing a base material sleeve (50) including a first wire member (29) formed of a high-strength fiber and a second wire member (30) formed of a fusible material; and
a heating and cutting step of heating and cutting multiple spots of the base material sleeve (50) in an axial direction and preparing the reinforcement member (28, 28a) in which ring-shaped inflation restriction portions (36) that are formed of fusible materials and are welded to the first wire member (29) are respectively formed at both end portions in the axial direction.

7. The catheter manufacturing method according to Claim 6,
wherein in the base material sleeve preparation step, a state where the multiple second wire members (30) are disposed along a tubularly net-shaped body (37) formed of the first wire member (29) so as to be spaced in a circumferential direction of the tubularly net-shaped body (37) is established.

8. The catheter manufacturing method according to Claim 7,
wherein in the base material sleeve preparation step, outer sides of the multiple second wire members (30) disposed along an outer surface of a mandrel are covered with the tubularly net-shaped body (37).

9. The catheter manufacturing method according to Claim 6,
wherein in the base material sleeve preparation step, the first wire member (29) and the second wire member (30) form the tubularly net-shaped base material sleeve (50).

10. The catheter manufacturing method according to Claim 9,
wherein in the base material sleeve preparation step, the base material sleeve (50) is formed such that a gross-sectional area of a cross-section perpendicular to the axial direction of the base material sleeve (50) in the first wire member (29) is greater than a gross-sectional area of that in the second wire member (30).
